# EUROPÄISCHE PATENTSCHRIFT

(11) **EP 4 554 905 B1**
(45) Veröffentlichungstag und Bekanntmachung des Hinweises auf die Patenterteilung: **20.05.2026**
(21) Anmeldenummer: 23761886.3
(22) Anmeldetag: 25.08.2023
(51) Int. Cl.: C02F 11/12, A61L 9/014, A61L 9/20

(54) **VORRICHTUNG ZUM TROCKNEN VON MATERIAL, INSBESONDERE BIOMASSE, BEISPIELSWEISE KLÄRSCHLAMM**
DEVICE FOR DRYING MATERIAL, IN PARTICULAR BIOMASS, FOR EXAMPLE SEWAGE SLUDGE
DISPOSITIF DE SÉCHAGE DE MATIÈRE, EN PARTICULIER DE BIOMASSE, PAR EXEMPLE DE BOUES D'ÉPURATION

(30) Priorität: 02.09.2022 DE 202022104976 U
(43) Veröffentlichungstag der Anmeldung: 21.05.2025
(73) Patentinhaber: Hellmuth, Felix, 96450 Coburg (DE)
(72) Erfinder: Hellmuth, Felix, 96450 Coburg (DE)
(74) Vertreter: Meissner Bolte Nürnberg
(86) Internationale Anmeldenummer: PCT/EP2023/073349
(87) Internationale Veröffentlichungsnummer: WO 2024/046901

(56) Entgegenhaltungen:
- DE-U1- 202021 102 132
- DATABASE WPI Week 201856, Derwent World Patents Index; AN 2018-64601B, XP002810443
- DATABASE WPI Week 201438, Derwent World Patents Index; AN 2014-F98274, XP002810445
- DATABASE WPI Week 200914, Derwent World Patents Index; AN 2009-E67363, XP002810447
- DATABASE WPI Week 200948, Derwent World Patents Index; AN 2009-L55744, XP002810444
- DATABASE WPI Week 200949, Derwent World Patents Index; AN 2009-L55947, XP002810446

## Beschreibung

Die Erfindung betrifft eine Vorrichtung zum Trocknen von Material, insbesondere Biomasse, beispielsweise Klärschlamm. Es kann sich bei der Biomasse auch um Milchschlamm, Biertreber, Gärreste, Gülle, Tiergülle oder Biogasgülle handeln. Bei dem Material kann es sich auch um befeuchtete oder Feuchtigkeit enthaltende Masse oder Schlamm oder befeuchtete oder Feuchtigkeit enthaltende Teile oder Granulate, wie etwa Metallspänen oder Hackschnitzel handeln.

Bekannte Vorrichtungen bringen beispielsweise Klärschlamm zur Trocknung auf einer Trocknungsfläche aus. Diese Vorrichtungen weisen Fördereinrichtungen auf, mit denen der Klärschlamm auf der Trocknungsfläche verteilt bzw. über die Trocknungsfläche gefördert wird. Es ist ferner bekannt, mehrere Trocknungsflächen übereinander anzuordnen. Ein bekanntes Problem bei derartigen Vorrichtungen besteht darin, dass es in Trocknungsvorrichtungen, insbesondere bei höheren Temperaturen, zu erheblicher Geruchsentwicklung kommen kann.

Eine Vorrichtung zum Trocknen von insbesondere Klärschlamm, gemäß dem Oberbegriff des Anspruchs 1 ist aus der DE 20 2021 102 132 U1 bekannt.

Der Erfindung liegt die Aufgabe zugrunde, eine neue Vorrichtung zum Trocknen von Material anzugeben. Insbesondere soll eine Vorrichtung vorgeschlagen werden, bei der die Geruchsintensität der Abluft der Vorrichtung vermindert ist und/oder Partikel aus der Abluft gefiltert werden und/oder eine Desinfizierung und/oder Entkeimung der Abluft erfolgt.

Diese Aufgabe wird gelöst durch eine Vorrichtung mit den Merkmalen des Anspruchs 1. Vorteilhafte Ausgestaltungen und Weiterbildungen sind in den abhängigen Ansprüchen angegeben.

Die erfindungsgemäße Vorrichtung zum Trocknen von Material, insbesondere Biomasse, beispielsweise Klärschlamm, umfasst ein Gehäuse mit wenigstens einer Trocknungskammer und einer Abluftkammer, wobei die Abluftkammer eine oder mehrere Luftabführungen aufweist. Die Erfindung ist dadurch gekennzeichnet, dass zwischen der Trocknungskammer und der Abluftkammer ein Abluftfiltersystem, insbesondere zur Partikel- und/oder Geruchsfilterung und/oder zur Desinfizierung und/oder Entkeimung, angeordnet ist.

Der Vorteil des Abluftfiltersystems liegt insbesondere in der Geruchsfilterung. Mit der erfindungsgemäßen Vorrichtung ist es möglich, die Geruchsintensität der Abluft maßgeblich zu verringern. Ein weiterer Vorteil liegt in der Partikelfilterung und in der Desinfizierung und/oder Entkeimung der Abluft.

Das Abluftfiltersystem ist in einer die Trocknungskammer und die Abluftkammer separierenden Trennwand angeordnet.

Gemäß einer Ausführungsvariante der Erfindung umfasst das Abluftfiltersystem ein oder mehrere UV-Lampen, vorzugsweise UV-C-Lampen, wobei die UV-Lampen insbesondere zum Desinfizieren und/oder Entkeimen der Abluft vorgesehen sind. Neben der Desinfizierung und Entkeimung der Abluft durch die von den UV-Lampen ausgehenden UV-Strahlung kann hierdurch auch eine Geruchsreduktion durch Oxidation, beispielsweise von Ammoniak, erreicht werden.

Das Abluftfiltersystem kann ein oder mehrere Partikelfilter umfassen, wobei der oder die Partikelfilter insbesondere jeweils durch ein Vlies ausgebildet sind. Die Partikelfilter können Grobstaubfilter und/oder Feinstaubfilter sein oder umfassen.

Ferner kann das Abluftfiltersystem einen oder mehrere Aktivkohlefilter umfassen. Der oder die Partikelfilter sind dem oder den Aktivkohlefiltern in Strömungsrichtung der Abluft insbesondere vorgeschalten. Die Strömungsrichtung der Abluft ist insbesondere von der Trocknungskammer zur Abluftkammer und/oder den Luftabführungen gerichtet.

Mit einem oder mehreren Partikelfiltern, insbesondere mehreren Partikelfiltern für unterschiedliche Partikelgrößen, lassen sich geruchsinfizierte Partikel herausfiltern und Gerüche deutlich abmildern. Falls dies nicht ausreichen sollte, ist zusätzlich ein Aktivkohlefilter zweckmäßig, der zumindest einen großen Teil der Gerüche aufnimmt.

Auch kann vorgesehen sein, dass die UV-Lampe oder die UV-Lampen oder eine oder mehrere der UV-Lampen in Strömungsrichtung der Abluft zwischen dem oder den Partikelfiltern und dem oder den Aktivkohlefiltern angeordnet sind.

Es kann vorgesehen sein, dass das Abluftfiltersystem ein Einschubfiltersystem mit ein oder mehreren, insbesondere ein bis acht, beispielsweise vier, auswechselbaren Filtereinschüben ist, wobei jeder Filtereinschub beispielsweise mit einem oder mehreren Partikelfiltern und/oder einem oder mehreren Aktivkohlefiltern und/oder einer oder mehreren UV-Lampen ausgestattet ist. Dies hat den Vorteil erhöhter Flexibilität. Die Vorrichtung kann dadurch hinsichtlich ihrer Abluft auch noch am jeweiligen Aufstellungsort ohne Aufwand und ohne hohe Investitionskosten hinsichtlich der konkreten Filteranforderungen, die sich beispielsweise im Rahmen einer Genehmigung oder Abnahme ergeben, durch Austausch oder Nachrüstung geeigneter Filtereinschübe angepasst werden. Bevorzugt ist das Einschubfiltersystem in der Trennwand angeordnet, wobei die Filtereinschübe in die Trennwand, insbesondere in Ausnehmungen in der Trennwand, einschiebbar sind. Beispielsweise kann jeder Filtereinschub in Strömungsrichtung der Abluft eingangsseitig einen oder mehrere Partikelfilter und ausgangsseitig einen oder mehrere Aktivkohlefilter aufweisen, wobei zwischen dem oder den Partikelfiltern und dem oder den Aktivkohlefiltern ein oder mehrere UV-Lampen angeordnet sind.

Die UV-Lampe oder die UV-Lampen oder eine oder mehrere der UV-Lampen können derart angeordnet sein, dass die UV-Lampen in die Trocknungskammer, insbesondere in den Bereich der Trocknungskammer vor der Trennwand, strahlen. Gemäß einer Weiterbildung ist diese UV-Lampe oder sind diese UV-Lampen an der hin zur Trocknungskammer orientierten Seite jedes Filtereinschubs angeordnet.

Eine Ausführungsvariante der Erfindung sieht vor, dass die Luftabführung einen Luftabführ-Ventilator zur Unterstützung der Luftabführung aus dem Gehäuse aufweist. Dieser Luftabfuhr-Ventilator sorgt für Luftströmungen in der Vorrichtung, und insbesondere in der Abluftkammer, und ebenfalls für die Abführung der bei der Trocknung mit Feuchtigkeit angereicherten Luft, wodurch die Trocknung beschleunigt wird.

Bevorzugt ist angrenzend an den oder die Luftabführungen außen am Gehäuse ein Kaminschacht, insbesondere zur Abführung der Abluft in die Umgebung, angeordnet.

Zudem kann vorgesehen sein, dass das Gehäuse eine oder mehrere Luftzuführungen aufweist, die zusammen mit der oder den Luftabführungen derart angeordnet sind, dass die Luft zum Trocknen des Materials durch die Vorrichtung geführt wird.

Gemäß der Erfindung weist die Abluftkammer eine Tür auf, wobei die Abluftkammer mittels der Tür von außerhalb des Gehäuses aus betreten werden kann oder betretbar ist. Dies ermöglicht beispielsweise Servicepersonal einen einfachen Zugang zum Abluftfiltersystem, beispielsweise zu Wartungszwecken und/oder zum Austausch oder zum Nachrüsten von Filterelementen und/oder Filtereinschüben.

In der Trocknungskammer sind ein oder mehrere Trocknungsflächen-Paare angeordnet, wobei jedes Trocknungsflächen-Paar eine obere Trocknungsfläche und eine untere Trocknungsfläche aufweist, über die das Material zum Trocknen förderbar ist, wobei jedem Trocknungsflächen-Paar eine um die obere Trocknungsfläche umlaufende Fördereinrichtung mit Förderelementen zum Fördern des Materials über die obere Trocknungsfläche und die untere Trocknungsfläche zugeordnet ist, wobei die obere Trocknungsfläche und die untere Trocknungsfläche sowie die Fördereinrichtung und deren Förderelemente derart ausgebildet und zueinander angeordnet sind, dass die Förderelemente zum Fördern des Materials über die obere Trocknungsfläche und zum Fördern des Materials über die untere Trocknungsfläche in entgegengesetzte Richtungen bewegt werden, wobei die obere Trocknungsfläche derart relativ zur unteren Trocknungsfläche angeordnet ist, dass das Material nach der Förderung über die obere Trocknungsfläche zur anschließenden Förderung über die untere Trocknungsfläche von der oberen Trocknungsfläche auf die untere Trocknungsfläche fällt.

Es kann vorgesehen sein, dass mindestens vier oder mindestens acht oder mindestens sechzehn Trocknungsflächen-Paare derart übereinander angeordnet sind, dass das Material nacheinander über alle Trocknungsflächen förderbar ist oder gefördert wird und hierbei von der unteren Trocknungsfläche des jeweils oberen Trocknungsflächen-Paares auf die obere Trocknungsfläche des jeweils unteren Trocknungsflächen-Paares fällt, bis die untere Trocknungsfläche des untersten Trocknungsflächen-Paares erreicht ist.

Die Erfindung wird nachstehend auch hinsichtlich weiterer Merkmale und Vorteile anhand der Beschreibung von Ausführungsbeispielen und unter Bezugnahme auf die beiliegenden, schematischen Zeichnungen näher erläutert. Es zeigen
- FIG. 1: ein Ausführungsbeispiel einer erfindungsgemäßen Vorrichtung zum Trocknen in einer schematischen, dreidimensionalen Schnittdarstellung,
- FIG. 2: das Ausführungsbeispiel nach FIG. 1 in einer weiteren schematischen, dreidimensionalen Schnittdarstellung, ohne Darstellung des Abluftfiltersystems, und
- FIG. 3: ein weiteres Ausführungsbeispiel einer erfindungsgemäßen Vorrichtung zum Trocknen in einer schematischen, dreidimensionalen Schnittdarstellung.

Einander entsprechende Teile und Komponenten sind in den Figuren mit den gleichen Bezugszeichen versehen.

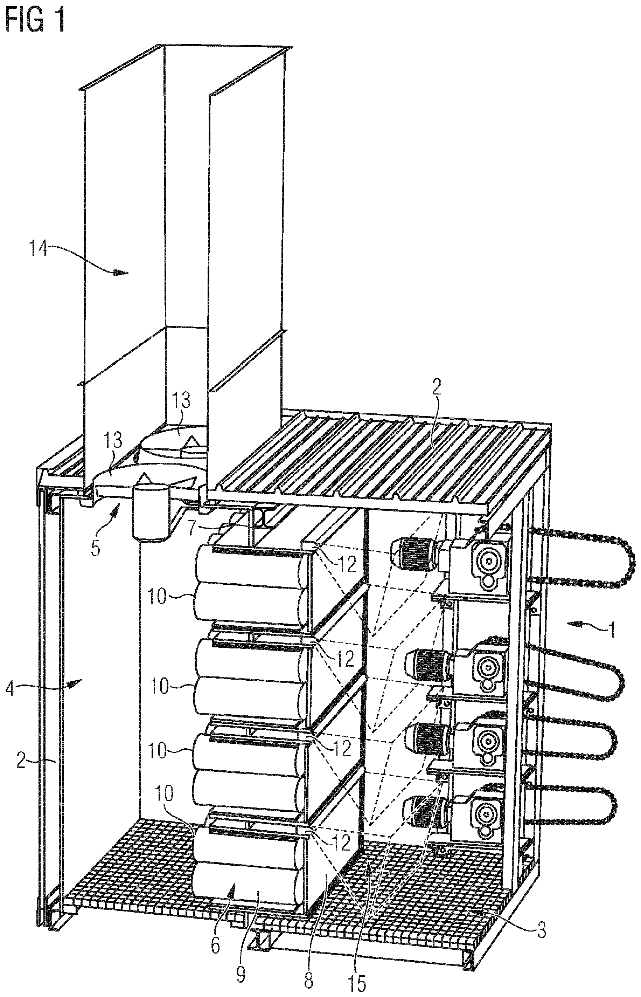

FIG 1 zeigt eine Vorrichtung 1 zum Trocknen von Biomasse, beispielsweise Klärschlamm. Die Vorrichtung 1 umfasst ein Gehäuse 2 mit einer Trocknungskammer 3 und einer Abluftkammer 4, wobei die Trocknungskammer in FIG. 1 lediglich teilweise dargestellt ist. Gemäß der Darstellung nach FIG. 1 ist die Vorrichtung sowohl längs als auch quer durch die Trocknungskammer 3 geschnitten.

In der Trocknungskammer 3 sind mehrere (nicht in den Figuren dargestellte) Trocknungsflächen-Paare angeordnet, wobei jedes Trocknungsflächen-Paar eine obere Trocknungsfläche und eine untere Trocknungsfläche aufweist, über die das Material zum Trocknen förderbar ist. Jedem Trocknungsflächen-Paar ist eine um die obere Trocknungsfläche umlaufende Fördereinrichtung mit Förderelementen zum Fördern des Materials über die obere Trocknungsfläche und die untere Trocknungsfläche zugeordnet, wobei die obere Trocknungsfläche und die untere Trocknungsfläche sowie die Fördereinrichtung und deren Förderelemente derart ausgebildet und zueinander angeordnet sind, dass die Förderelemente zum Fördern des Materials über die obere Trocknungsfläche und zum Fördern des Materials über die untere Trocknungsfläche in entgegengesetzte Richtungen bewegt werden. Die obere Trocknungsfläche ist derart relativ zur unteren Trocknungsfläche angeordnet, dass das Material nach der Förderung über die obere Trocknungsfläche zur anschließenden Förderung über die untere Trocknungsfläche von der oberen Trocknungsfläche auf die untere Trocknungsfläche fällt. Es können mindestens vier, acht oder auch sechzehn Trocknungsflächen-Paare derart übereinander angeordnet sein, dass das Material nacheinander über alle Trocknungsflächen gefördert wird und hierbei von der unteren Trocknungsfläche des jeweils oberen Trocknungsflächen-Paares auf die obere Trocknungsfläche des jeweils unteren Trocknungsflächen-Paares fällt, bis die untere Trocknungsfläche des untersten Trocknungsflächen-Paares erreicht ist.

Die Abluftkammer 4 weist zwei Luftabführungen 5 auf. Jede Luftabführung 5 weist einen Luftabführ-Ventilator 13 zur Unterstützung der Luftabführung aus dem Gehäuse 2 auf. Angrenzend an die Luftabführungen 5 ist außen am Gehäuse 2 ein Kaminschacht 14 angeordnet. Die Abluftkammer 4 weist eine (nicht in den Figuren dargestellte) Tür auf, wobei die Abluftkammer 4 mittels der Tür, beispielsweise zu Wartungszwecken wie einem Filterwechsel, von außerhalb des Gehäuses 2 aus betreten werden kann.

Zwischen der Trocknungskammer 3 und der Abluftkammer 4 ist ein Abluftfiltersystem 6 zur Partikel- und/oder Geruchsfilterung angeordnet, wobei das Abluftfiltersystem 6 in einer die Trocknungskammer 3 und die Abluftkammer 4 separierenden Trennwand 7 angeordnet ist.

Das Abluftfiltersystem 6 umfasst mehrere Partikelfilter 8, wobei die Partikelfilter 8 jeweils durch ein Vlies ausgebildet sind. Die Partikelfilter 8 können jeweils sowohl Grobstaubfilter als auch Feinstaubfilter umfassen. Ferner umfasst das Abluftfiltersystem 6 auch Aktivkohlefilter 9, wobei die Partikelfilter 8 den Aktivkohlefiltern 9 in Strömungsrichtung der Abluft vorgeschalten sind. Die Strömungsrichtung der Abluft ist von der Trocknungskammer 3 zur Abluftkammer 4 bzw. den Luftabführungen 5 gerichtet.

Das Abluftfiltersystem 6 ist ein Einschubfiltersystem mit vier auswechselbaren Filtereinschüben 10, wobei jeder Filtereinschub 10 mit einem Partikelfilter 8 und einem Aktivkohlefilter 9 ausgestattet ist. Das Einschubfiltersystem ist in der Trennwand 7 angeordnet, wobei die Filtereinschübe 10 in Ausnehmungen 11 in der Trennwand 7 einschiebbar sind.

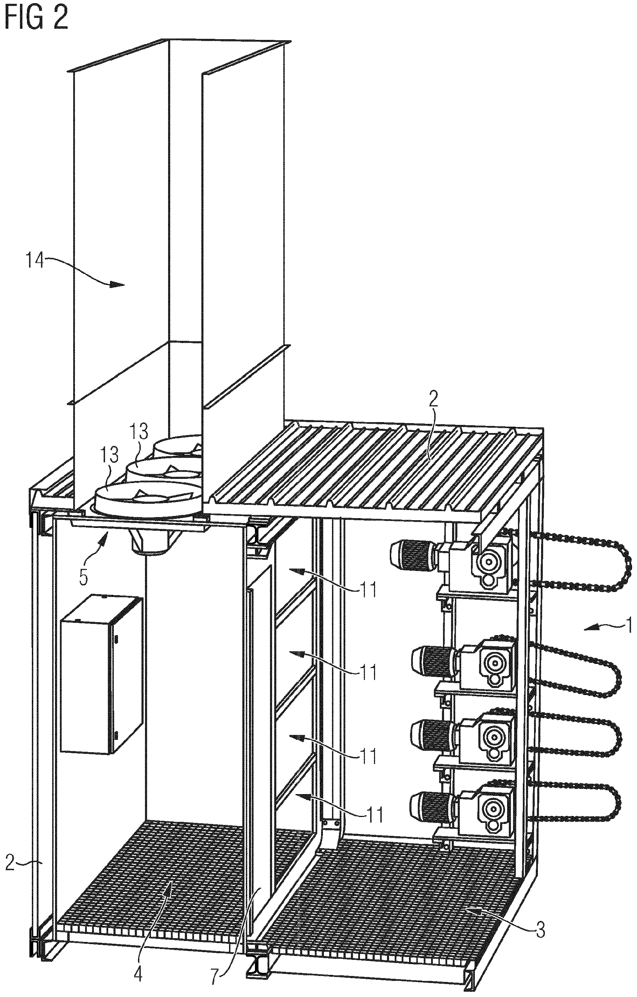

FIG. 2 zeigt die Vorrichtung 1 ohne das Abluftfiltersystem 6, wobei die Ausnehmungen 11 in der Trennwand 7 zu erkennen sind. Diese Ausnehmungen 11 sind zur Aufnahme der Filtereinschübe 10 ausgebildet und vorgesehen.

Wie FIG. 1 zeigt, umfasst das Abluftfiltersystem 6 mehrere UV-C-Lampen 12, wobei die UV-Lampen 12 zum Desinfizieren und/oder Entkeimen der Abluft vorgesehen sind. Die UV-Lampen 12 sind derart angeordnet, dass die UV-Lampen 12 in den Bereich 15 der Trocknungskammer 3 vor der Trennwand 7 strahlen. Es ist jeweils eine UV-Lampe 12 im oberen Bereich der hin zur Trocknungskammer 3 orientierten Seite jedes Filtereinschubs 10 angeordnet.

Das Gehäuse 2 weist ferner (nicht in den Figuren dargestellte) Luftzuführungen auf, die zusammen mit den Luftabführungen 5 derart angeordnet sind, dass die Luft zum Trocknen des Materials durch die Vorrichtung 1 und insbesondere die Trocknungskammer 3 sowie die Abluftkammer 4 geführt wird.

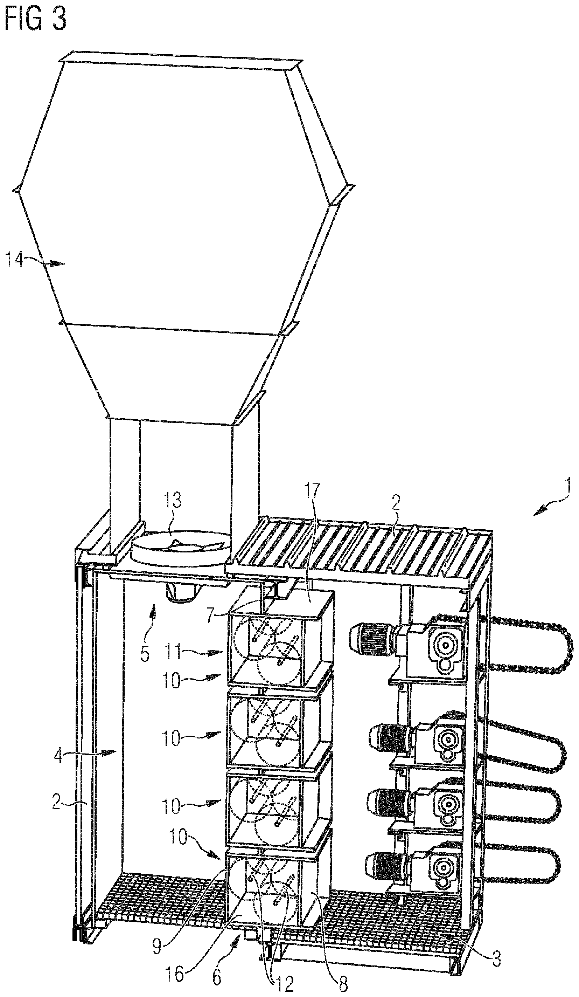

FIG. 3 zeigt ein weiteres, alternatives Ausführungsbeispiel einer erfindungsgemäßen Vorrichtung 1, wobei hinsichtlich der Übereinstimmungen auf die vorstehenden Ausführungen zu FIG. 1 und FIG. 2 verwiesen wird. In diesem Ausführungsbeispiel sind die Ausnehmungen 11 in der Trennwand 7 als Schächte 17 in der Trennwand ausgebildet. Diese hier als Schächte 17 ausgebildeten Ausnehmungen 11 sind zur Aufnahme der Filtereinschübe 10 ausgebildet und vorgesehen. Jeder Filtereinschub 10 weist in Strömungsrichtung der Abluft eingangsseitig einen oder mehrere Partikelfilter 8, beispielsweise einen oder mehrere Vliesfilter, und ausgangsseitig einen oder mehrere Aktivkohlefilter 9 auf. Zwischen dem oder den Partikelfiltern 8 und dem oder den Aktivkohlefiltern 9 sind in jedem Filtereinschub 10 mehrere UV-Lampen 12 angeordnet. Jeder Schacht 17 oder jeder Filtereinschub 10 ist, zumindest zwischen dem oder den Partikelfiltern 8 und dem oder den Aktivkohlefiltern 9, mit einer Auskleidung 16 versehen, die den Austritt von UV-Strahlung, die von den UV-Lampen ausgeht, verhindert. Beispielsweise kann es sich bei dieser Auskleidung 16 um eine Blechauskleidung handeln. Die UV-Lampen 12 bestrahlen somit die Abluft in einem abgedeckten Raum.

### Bezugszeichenliste

- 1: Vorrichtung
- 2: Gehäuse
- 3: Trocknungskammer
- 4: Abluftkammer
- 5: Luftabführung
- 6: Abluftfiltersystem
- 7: Trennwand
- 8: Partikelfilter
- 9: Aktivkohlefilter
- 10: Filtereinschub
- 11: Ausnehmung
- 12: UV-Lampe
- 13: Luftabführ-Ventilator
- 14: Kaminschacht
- 15: Bereich der Trocknungskammer 3 vor der Trennwand 7
- 16: Auskleidung des Filtereinschubs 10
- 17: Schacht in der Trennwand 7 für Filtereinschub 10

## Patentansprüche

1. Vorrichtung (1) zum Trocknen von Material umfassend
ein Gehäuse (2) mit wenigstens einer Trocknungskammer (3) und einer Abluftkammer (4),
wobei die Abluftkammer (4) eine oder mehrere Luftabführungen (5) aufweist, wobei zwischen der Trocknungskammer (3) und der Abluftkammer (4) ein Abluftfiltersystem (6) angeordnet ist,
wobei in der Trocknungskammer (3) ein oder mehrere Trocknungsflächen-Paare angeordnet sind, wobei jedes Trocknungsflächen-Paar eine obere Trocknungsfläche und eine untere Trocknungsfläche aufweist, über die das Material zum Trocknen förderbar ist,
wobei jedem Trocknungsflächen-Paar eine um die obere Trocknungsfläche umlaufende Fördereinrichtung mit Förderelementen zum Fördern des Materials über die obere Trocknungsfläche und die untere Trocknungsfläche zugeordnet ist,
wobei die obere Trocknungsfläche und die untere Trocknungsfläche sowie die Fördereinrichtung und deren Förderelemente derart ausgebildet und zueinander angeordnet sind, dass die Förderelemente zum Fördern des Materials über die obere Trocknungsfläche und zum Fördern des Materials über die untere Trocknungsfläche in entgegengesetzte Richtungen bewegt werden, und
wobei die obere Trocknungsfläche derart relativ zur unteren Trocknungsfläche angeordnet ist, dass das Material nach der Förderung über die obere Trocknungsfläche zur anschließenden Förderung über die untere Trocknungsfläche von der oberen Trocknungsfläche auf die untere Trocknungsfläche fällt, und wobei das Abluftfiltersystem (6) in einer die Trocknungskammer (3) und die Abluftkammer (4) separierenden Trennwand (7) angeordnet ist,
**dadurch gekennzeichnet,**
**dass** die Abluftkammer (4) eine Tür aufweist, wobei die Abluftkammer (4) mittels der Tür von außerhalb des Gehäuses (2) aus betreten werden kann oder betretbar ist.

2. Vorrichtung (1) nach Anspruch 1,
**dadurch gekennzeichnet, dass**
das Abluftfiltersystem (6) ein oder mehrere UV-Lampen (12), vorzugsweise UV-C-Lampen, umfasst, wobei die UV-Lampen (12) insbesondere zum Desinfizieren und/oder Entkeimen der Abluft vorgesehen sind.

3. Vorrichtung (1) nach einem der vorhergehenden Ansprüche,
**dadurch gekennzeichnet, dass**
das Abluftfiltersystem (6) ein oder mehrere Partikelfilter (8) umfasst, wobei der oder die Partikelfilter (8) insbesondere jeweils durch ein Vlies ausgebildet sind.

4. Vorrichtung (1) nach einem der vorhergehenden Ansprüche,
**dadurch gekennzeichnet, dass**
das Abluftfiltersystem (6) einen oder mehrere Aktivkohlefilter (9) umfasst.

5. Vorrichtung (1) nach den Ansprüchen 3 und 4,
**dadurch gekennzeichnet, dass**
der oder die Partikelfilter (8) dem oder den Aktivkohlefiltern (9) in Strömungsrichtung der Abluft vorgeschalten sind.

6. Vorrichtung (1) nach Anspruch 5 und nach Anspruch 2,
**dadurch gekennzeichnet, dass**
die UV-Lampe (12) oder die UV-Lampen (12) oder eine oder mehrere der UV-Lampen (12) in Strömungsrichtung der Abluft zwischen dem oder den Partikelfiltern (8) und dem oder den Aktivkohlefiltern (9) angeordnet sind.

7. Vorrichtung (1) nach einem der vorhergehenden Ansprüche,
**dadurch gekennzeichnet, dass**
das Abluftfiltersystem (6) ein Einschubfiltersystem mit einem oder mehreren, insbesondere ein bis acht, beispielsweise vier, auswechselbaren Filtereinschüben (10) ist, wobei jeder Filtereinschub (10) beispielsweise mit einem oder mehreren Partikelfiltern (8) und/oder einem oder mehreren Aktivkohlefiltern (9) und/oder einer oder mehreren UV-Lampen (12) ausgestattet ist.

8. Vorrichtung (1) nach Anspruch 7,
**dadurch gekennzeichnet, dass**
das Einschubfiltersystem in der Trennwand (7) angeordnet ist, wobei die Filtereinschübe (10) in die Trennwand (7), insbesondere in Ausnehmungen (11) in der Trennwand (7), einschiebbar sind.

9. Vorrichtung (1) nach einem der vorhergehenden Ansprüche und nach Anspruch 2,
**dadurch gekennzeichnet, dass**
die UV-Lampe (12) oder die UV-Lampen (12) oder eine oder mehrere der UV-Lampen (12) derart angeordnet sind, dass die UV-Lampen (12) in die Trocknungskammer (3), insbesondere in den Bereich (15) der Trocknungskammer (3) vor der Trennwand (7), strahlen.

10. Vorrichtung (1) nach Anspruch 9 und einem der Ansprüche 7 oder 8,
**dadurch gekennzeichnet, dass**
die im die Trocknungskammer (3) strahlende UV-Lampe (12) oder die in die Trocknungskammer (3) strahlenden UV-Lampen (12) an der hin zur Trocknungskammer (3) orientierten Seite jedes Filtereinschubs (10) angeordnet sind.

11. Vorrichtung (1) nach einem der vorhergehenden Ansprüche,
**dadurch gekennzeichnet, dass**
die Luftabführung (5) einen Luftabführ-Ventilator (13) zur Unterstützung der Luftabführung aus dem Gehäuse (2) aufweist.

12. Vorrichtung (1) nach einem der vorhergehenden Ansprüche,
**dadurch gekennzeichnet, dass**
angrenzend an den oder die Luftabführungen (5) außen am Gehäuse (2) ein Kaminschacht (14) angeordnet ist.

13. Vorrichtung (1) nach einem der vorhergehenden Ansprüche,
**dadurch gekennzeichnet, dass**
das Gehäuse (2) eine oder mehrere Luftzuführungen aufweist, die zusammen mit der oder den Luftabführungen (5) derart angeordnet sind, dass die Luft zum Trocknen des Materials durch die Vorrichtung (1) geführt wird.

14. Vorrichtung (1) nach einem der vorhergehenden Ansprüche,
**dadurch gekennzeichnet, dass**
mindestens vier oder mindestens acht oder mindestens sechzehn Trocknungsflächen-Paare derart übereinander angeordnet sind, dass das Material nacheinander über alle Trocknungsflächen förderbar ist oder gefördert wird und hierbei von der unteren Trocknungsfläche des jeweils oberen Trocknungsflächen-Paares auf die obere Trocknungsfläche des jeweils unteren Trocknungsflächen-Paares fällt, bis die untere Trocknungsfläche des untersten Trocknungsflächen-Paares erreicht ist.

## Claims

1. Apparatus (1) for drying material, comprising
a housing (2) with at least one drying chamber (3) and an exhaust air chamber (4),
wherein the exhaust air chamber (4) has one or more air outlets (5),
wherein an exhaust air filter system (6) is arranged between the drying chamber (3) and the exhaust air chamber (4),
wherein one or more pairs of drying surfaces are arranged in the drying chamber (3), wherein each pair of drying surfaces comprises an upper drying surface and a lower drying surface over which the material can be conveyed for drying,
wherein each pair of drying surfaces is associated with a conveyor device encircling the upper drying surface, comprising conveyor elements for conveying the material over the upper drying surface and the lower drying surface, wherein the upper drying surface and the lower drying surface, as well as the conveying device and its conveying elements, are designed and arranged relative to one another such that the conveying elements are moved in opposite directions for conveying the material over the upper drying surface and for conveying the material over the lower drying surface, and
wherein the upper drying surface is arranged relative to the lower drying surface such that, after being conveyed over the upper drying surface, the material falls from the upper drying surface onto the lower drying surface for subsequent conveyance over the lower drying surface, and
wherein the exhaust air filter system (6) is arranged in a partition wall (7) separating the drying chamber (3) and the exhaust air chamber (4), **characterised in**
**that** the exhaust air chamber (4) comprises a door, whereby the exhaust air chamber (4) can be accessed from outside the housing (2) via the door.

2. Apparatus (1) according to claim 1,
**characterised in that**
the exhaust air filter system (6) comprises one or more UV lamps (12), preferably UV-C lamps, wherein the UV lamps (12) are provided in particular for disinfecting and/or sterilising the exhaust air.

3. Apparatus (1) according to one of the preceding claims,
**characterised in that**
the exhaust air filter system (6) comprises one or more particle filters (8), wherein the particle filter(s) (8) are each formed in particular by a fleece.

4. Apparatus (1) according to one of the preceding claims,
**characterised in that**
the exhaust air filter system (6) comprises one or more activated carbon filters (9).

5. Apparatus (1) according to claims 3 and 4,
**characterised in that**
the particle filter(s) (8) are arranged upstream of the activated carbon filter(s) (9) in the direction of flow of the exhaust air.

6. Apparatus (1) according to claim 5 and claim 2,
**characterised in that**
the UV lamp (12) or the UV lamps (12) or one or more of the UV lamps (12) are arranged in the direction of flow of the exhaust air between the particle filter(s) (8) and the activated carbon filter(s) (9).

7. Apparatus (1) according to one of the preceding claims,
**characterised in that**
the exhaust air filter system (6) is a plug-in filter system comprising one or more, in particular one to eight, for example four, replaceable filter inserts (10), wherein each filter insert (10) is equipped, for example, with one or more particle filters (8) and/or one or more activated carbon filters (9) and/or one or more UV lamps (12).

8. Apparatus (1) according to claim 7,
**characterised in that**
the plug-in filter system is arranged in the partition wall (7), wherein the filter inserts (10) can be inserted into the partition wall (7), in particular into recesses (11) in the partition wall (7).

9. Apparatus (1) according to one of the preceding claims and according to claim 2,
**characterised in that**
the UV lamp (12) or the UV lamps (12) or one or more of the UV lamps (12) are arranged such that the UV lamps (12) irradiate the drying chamber (3), in particular the region (15) of the drying chamber (3) in front of the partition wall (7).

10. Apparatus (1) according to claim 9 and one of claims 7 or 8,
**characterised in that**
the UV lamp (12) or UV lamps (12) emitting light into the drying chamber (3) are arranged on the side of each filter insert (10) facing the drying chamber (3).

11. Apparatus (1) according to one of the preceding claims,
**characterised in that**
the air outlet (5) comprises an air exhaust fan (13) to assist in the extraction of air from the housing (2).

12. Apparatus (1) according to one of the preceding claims,
**characterised in that**
a chimney shaft (14) is arranged on the outside of the housing (2) adjacent to the air outlet(s) (5).

13. Apparatus (1) according to one of the preceding claims,
**characterised in that**
the housing (2) comprises one or more air inlets which, together with the air outlet(s) (5), are arranged such that the air for drying the material is guided through the apparatus (1).

14. Apparatus (1) according to one of the preceding claims,
**characterised in that**
at least four, or at least eight, or at least sixteen pairs of drying surfaces are arranged one above the other in such a way that the material can be or is conveyed successively over all the drying surfaces, falling in the process from the lower drying surface of the respective upper pair of drying surfaces onto the upper drying surface of the respective lower pair of drying surfaces, until the lower drying surface of the lowest pair of drying surfaces is reached.

## Revendications

1. Dispositif (1) pour le séchage de matériaux, comprenant
un boîtier (2) comportant au moins une chambre de séchage (3) et une chambre d'évacuation d'air (4),
la chambre d'évacuation d'air (4) comportant une ou plusieurs évacuations d'air (5),
un système de filtration d'air évacué (6) étant disposé entre la chambre de séchage (3) et la chambre d'évacuation d'air (4),
dans laquelle une ou plusieurs paires de surfaces de séchage sont disposées dans la chambre de séchage (3), chaque paire de surfaces de séchage comportant une surface de séchage supérieure et une surface de séchage inférieure sur lesquelles le matériau peut être transporté pour être séché,
chaque paire de surfaces de séchage étant associée à un dispositif de transport s'étendant autour de la surface de séchage supérieure et comportant des éléments de transport destinés à transporter le matériau sur la surface de séchage supérieure et la surface de séchage inférieure,
la surface de séchage supérieure et la surface de séchage inférieure, ainsi que le dispositif de transport et ses éléments de transport, étant conçus et disposés les uns par rapport aux autres de telle sorte que les éléments de transport soient déplacés dans des directions opposées pour transporter le matériau sur la surface de séchage supérieure et pour transporter le matériau sur la surface de séchage inférieure, et
la surface de séchage supérieure étant disposée par rapport à la surface de séchage inférieure de telle sorte que, après avoir été transporté sur la surface de séchage supérieure, le matériau tombe de la surface de séchage supérieure sur la surface de séchage inférieure en vue de son transport ultérieur sur cette dernière, et
le système de filtration de l'air évacué (6) étant disposé dans une paroi de séparation (7) séparant la chambre de séchage (3) et la chambre d'air évacué (4),
caractérisé en ce
en ce que la chambre d'évacuation d'air (4) comporte une porte, la chambre d'évacuation d'air (4) pouvant être accessible depuis l'extérieur du boîtier (2) au moyen de la porte.

2. Dispositif (1) selon la revendication 1,
**caractérisé en ce que**
le système de filtration de l'air évacué (6) comprend une ou plusieurs lampes UV (12), de préférence des lampes UV-C, lesdites lampes UV (12) étant notamment destinées à désinfecter et/ou à stériliser l'air évacué.

3. Dispositif (1) selon l'une des revendications précédentes,
**caractérisé en ce que**
le système de filtration de l'air évacué (6) comprend un ou plusieurs filtres à particules (8), le ou les filtres à particules (8) étant notamment constitués chacun d'un non-tissé.

4. Dispositif (1) selon l'une des revendications précédentes,
**caractérisé en ce que**
le système de filtration de l'air évacué (6) comprend un ou plusieurs filtres à charbon actif (9).

5. Dispositif (1) selon les revendications 3 et 4,
**caractérisé en ce que**
le ou les filtres à particules (8) sont placés en amont du ou des filtres à charbon actif (9) dans le sens d'écoulement de l'air évacué.

6. Dispositif (1) selon la revendication 5 et selon la revendication 2,
**caractérisé en ce que**
la ou les lampes UV (12) sont disposées dans le sens d'écoulement de l'air évacué, entre le ou les filtres à particules (8) et le ou les filtres à charbon actif (9).

7. Dispositif (1) selon l'une des revendications précédentes,
**caractérisé en ce que**
le système de filtration de l'air évacué (6) est un système de filtres à cartouches comprenant une ou plusieurs, en particulier une à huit, par exemple quatre, cartouches filtrantes remplaçables (10), chaque cartouche filtrante (10) étant équipée, par exemple, d'un ou plusieurs filtres à particules (8) et/ou d'un ou plusieurs filtres à charbon actif (9) et/ou d'une ou plusieurs lampes UV (12).

8. Dispositif (1) selon la revendication 7,
**caractérisé en ce que**
le système de filtres amovibles est disposé dans la cloison (7), les cartouches filtrantes (10) pouvant être insérées dans la cloison (7), en particulier dans des évidements (11) de la cloison (7).

9. Dispositif (1) selon l'une des revendications précédentes et selon la revendication 2,
**caractérisée en ce que**
la lampe UV (12) ou les lampes UV (12) ou une ou plusieurs des lampes UV (12) sont disposées de telle sorte que les lampes UV (12) rayonnent dans la chambre de séchage (3), en particulier dans la zone (15) de la chambre de séchage (3) située en amont de la cloison (7).

10. Dispositif (1) selon la revendication 9 et l'une des revendications 7 ou 8,
**caractérisé en ce que**
la lampe UV (12) ou les lampes UV (12) émettant dans la chambre de séchage (3) sont disposées sur le côté de chaque tiroir filtrant (10) tourné vers la chambre de séchage (3).

11. Dispositif (1) selon l'une des revendications précédentes,
**caractérisé en ce que**
l'évacuation d'air (5) comporte un ventilateur d'évacuation d'air (13) destiné à faciliter l'évacuation de l'air hors du boîtier (2).

12. Dispositif (1) selon l'une des revendications précédentes,
**caractérisé en ce que**
un conduit de cheminée (14) est disposé à l'extérieur du boîtier (2), à proximité immédiate de la ou des évacuations d'air (5).

13. Dispositif (1) selon l'une des revendications précédentes,
**caractérisé en ce que**
le boîtier (2) comporte une ou plusieurs entrées d'air qui, conjointement avec la ou les évacuations d'air (5), sont disposées de telle sorte que l'air destiné au séchage du matériau soit acheminé à travers le dispositif (1).

14. Dispositif (1) selon l'une des revendications précédentes,
**caractérisé en ce que**
au moins quatre, au moins huit ou au moins seize paires de surfaces de séchage sont disposées les unes au-dessus des autres de telle sorte que le matériau puisse être ou soit acheminé successivement sur toutes les surfaces de séchage et tombe ainsi de la surface de séchage inférieure de la paire de surfaces de séchage supérieure vers la surface de séchage supérieure de la paire de surfaces de séchage inférieure, jusqu'à ce que la surface de séchage inférieure de la paire de surfaces de séchage la plus basse soit atteinte.
